# EUROPEAN PATENT SPECIFICATION

(11) **EP 3 441 095 B1**
(45) Date of publication and mention of the grant of the patent: **20.10.2021**
(21) Application number: 18196042.8
(22) Date of filing: 13.12.2007
(51) Int. Cl.: A61M 5/142, A61M 39/28, A61M 5/168, A61M 39/08

(54) **Administration set with two keys**
Verwaltungssatz mit zwei Schlüsseln
Ensemble d'administration à deux clés

(30) Priority: 25.12.2006 IL 18030506
(43) Date of publication of application: 13.02.2019
(62) Divisional of application: 07024175.7
(73) Proprietor: Caesarea Medical Electronics Ltd., 38900 Caesarea (IL)
(72) Inventor: Barak, Swi, 38900 Caesarea (IL)
(74) Representative: Harrison IP Limited

(56) References cited:
- EP-A- 1 247 538
- EP-A1- 1 938 851
- WO-A-2006/083933
- US-A- 4 798 590
- US-A1- 2005 277 890

## Description

### Field and background of the invention

Systems for administration of liquids to a patient are widely known. However, a variety of different pumps are available for propelling liquid to a patient, which may differ, among others, by construction and safety of use.

Flow sets for use with a liquid pump must be attentively designed for safe use. A dedicated pumping tube-segment of the flow set must be installed in the pump and the tube-segment must be installed in the right place, and held taut, straight and stretched only up to a determined value. The flow set must be full of liquid before using it and should remain full as long as it is in use.

Moreover, the tube-segment must be replaced by a new pumping tube-segment when it loses its flexibility.

US 4,798,590 discloses a flow set comprising a drip chamber, a flexible plastic line, having a roller clamp, an inlet connector coupled to at the end to a pump chamber, wherein the other end of the pump chamber is coupled to an outlet connector. The outlet connector is coupled through a line having a roller clamp and a Y-site therein to a luer.

EP 1 938 851 discloses a disposable flow set comprising: (a) a drip chamber or a spike; (b) a first administration tube; (c) at least one pumping tube-segment; (d) a second administration tube; (e) an anti-free-flow valve; (f) at least one identifying-key, whereby the or each pumping tube-segment has a respective identifying-key connected thereto and wherein each identifying-key comprises a hoop that is clamped on said respective tube-segment and includes: i. a number of teeth with a unique combination of location and width, ii. a pressing-plate (g) at least one stretching-key.

EP 1 247 538 discloses a disposal flow set comprising a drip chamber or a spike, a first administration tube to administrate liquid from said drip chamber, at least one pumping tube segment, connected to said first administration tube, a second administration tube in the end of the last of said pumping tube-segments and an anti-free-flow valve installed in the end of said second administration tube.

US 2005/277890 discloses a medical delivery system for delivering a medicament or fluid to a patient. The system comprises a disposable element such as a line set associated with a container containing the fluid, an identifier associated with the line set and having identification information associated therewith, and a delivery device configured to engage the line set and deliver the fluid to the patient. The delivery device includes a recognition system that is capable of obtaining the identification information associated with the identifier. The identification information may include information regarding the identification of the fluid, a type of line set, or a type of administration associated with the line set. The device can be capable of configuration based on the identification information.

WO 2006/083933 discloses a system and method for automatically delivering an infusate to a patient. The system includes an infusion set and an infusion device. A signalling component disposed on an infusion set component identifies an administration protocol for the infusion set. A detection device operatively connected to the infusion device detects the signalling component and identifies the administration protocol. The infusion device is then configured to operate according to the administration protocol.

### Summary of the invention

According to the present invention, there is provided a method of installing a pumping tube-segment in a liquid pump as hereinafter set forth in Claim 1 of the appended claims.

Preferred features of the invention are disclosed in the dependent claims.

### Brief description of the figures

The invention is herein described, by a way of example only, with reference to the accompanying drawing. With reference now to the specific detail in the drawings, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawing making it apparent to those skilled in the art how the several forms of the invention may be embodied in practice.

In the figures:
Figure 1 illustrates a flow set with a spike (11), a first administration tube-segment (12a), a pumping tube-segment (12b), a stretching-key (13), an identifying-key (14), a second administration tube-segment (12c) and an anti-free-flow valve (15),
Figure 2 illustrates the installation of a pumping tube segment (12b) in a liquid pump (21),
Figure 3 illustrates a cross-section of the identifying-key (14), and
Figure 4 illustrates a cross-section of the stretching-key (13).

### Description the preferred embodiment

The flow set of the present invention has a number of features that ensure the use of only a specific flow set with a compatible liquid pump. There is an identifying-key that prevents the insertion of a wrong flow set and enables the liquid pump to determine the presence of the compatible administration set. In addition, the flow set of the present invention has a number of features that ensure the use of the flow set in a way that prevents over stretching of the pumping tube-segment.

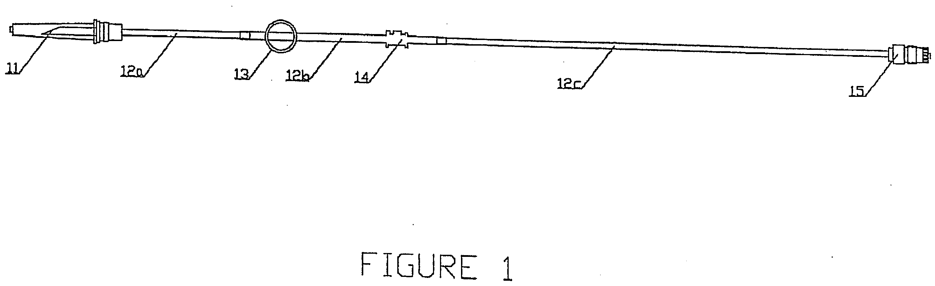

Referring now to Figure 1, that illustrates a flow set with a spike 11, a first administration tube-segment 12a, a pumping tube-segment 12b, a second administration tube 12c and an anti-free-flow valve 15. An identifying-key 14 is installed near one of the pumping tube-segment's ends. Following installation of the identifying-key 14 in a liquid pump 21 the stretching-key 13 is used to install the pumping tube-segment 12b in the liquid pump 21 in a correct position and stretched up to a determined degree.

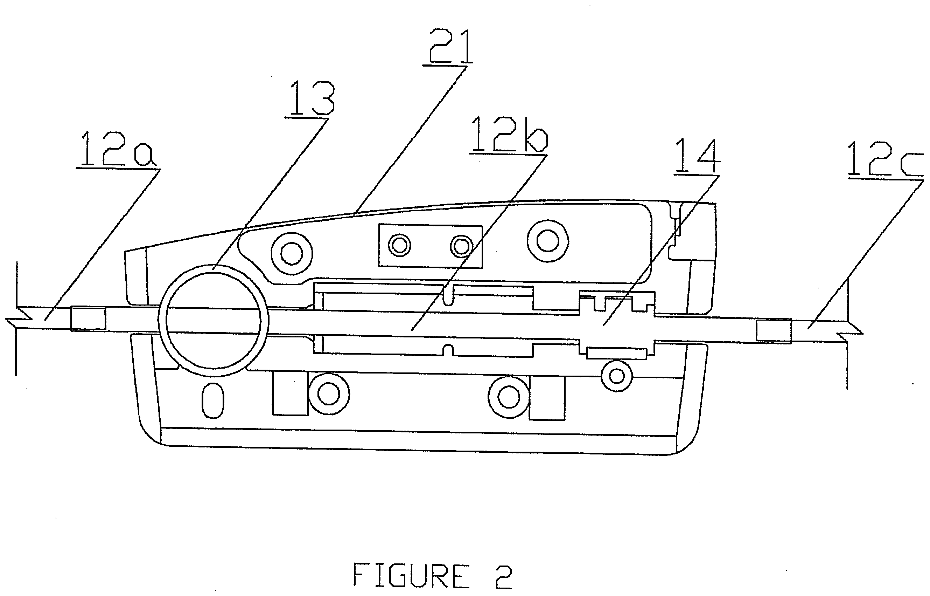

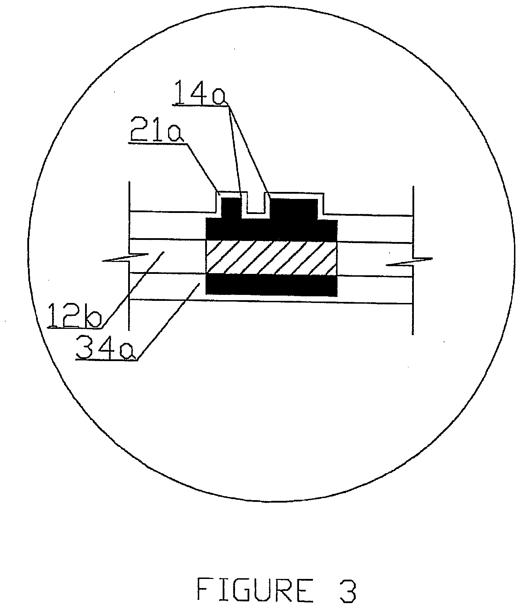

Figure 2 illustrates the way to install a pumping tube-segment 12b in a liquid pump 21. An identifying-key 14 is disposed on the pumping tube-segment 12b. The identifying-key 14 includes a collar that is clamped onto the tube-segment 12b and has a number of teeth 14a, shown in more detail in Figure 3. Each tooth has a specific width and specific location, creating a code that allows the inserting of the tube-segment 12b only to a specific liquid pump 21 that has a set of niches 21a that are arranged in accord with the same unique structural code.

The identifying-key 14 has also a pressure-plate 34a. This pressure-plate 34a is pressed by a door (not shown) of the liquid pump 21 and presses the pumping tube-segment 12b against a pressure-sensor (not shown) that is installed in the liquid pump 21 behind the identifying-key 14. The identifying-key 14 prevents the liquid pump 21 from starting operation unless the pressure-sensor measures a determined pressure. The stretching-key is designed to be installed in a recess 21b of the liquid pump 21. The determined distance between the identifying-key 14 and the stretching-key 13 corresponds to the respective distance between the identifying-key niches 21a and the stretching-key recess 21 b in the liquid pump 21, shown in more details in Figure 3. When both the identifying-key and the stretching-key are installed in the liquid pump 21, the determined distance between their locations on the pumping tube-segment 12b ensures that the pumping tube-segment 12b is tightened and held straight and stretched up to a determined degree.

Figure 3 illustrates a cross-section of an identifying-key 14. The identifying-key 14 is clamped onto the tube-segment 12b near one of its ends. The identifying-key 14 includes a set of teeth 14a to enable the insertion of the flow set only into a compatible liquid pump and presses the pumping tube-segment 12b against a pressure-sensor (not shown) of the liquid pump enabling the pressure-measuring.

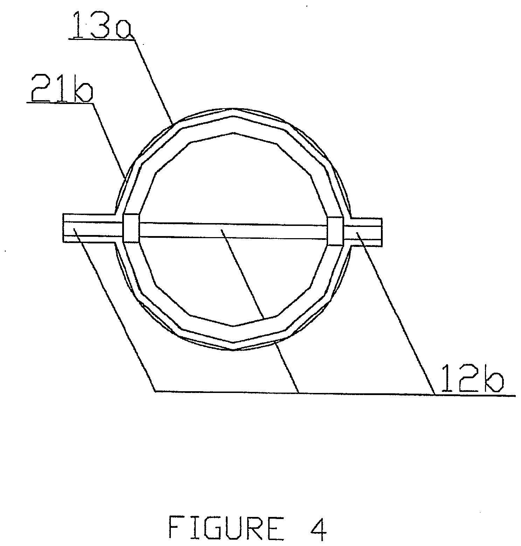

Figure 4 illustrates a cross-section of a stretching-key 13. The stretching-key 13 includes a collar clamped onto the pumping tube-segment 12b near its other end. The stretching-key 13 may be ring-shaped in order to enable an air sensor (not shown) to detect air in the tube-segment part located within the stretching-key centre, ensuring that the pumping tube-segment is placed in front of the air sensor for optimal detection of air in the pumping tube-segment.

Although the invention has been described in conjunction with specific embodiments thereof, it is evident that many alternatives, modifications and variations will be apparent to those skilled in the art, falling within the scope of the invention as defined by the appended claims.

## Claims

1. A method of installing a pumping tube-segment (12b) in a liquid pump (21), comprising:
providing the pumping tube segment (12b) having an identifying-key (14) disposed thereon, the identifying-key (14) including a collar that is clamped onto the pumping tube-segment (12b), the identifying-key (14) having a number of teeth (14a), each tooth having a specific width and specific location, thus creating a code that allows inserting of the pumping tube segment (12b) only to a specific liquid pump (21) that has a set of niches (21a) that are arranged in accord with the same unique structural code, the identifying-key (14) also including a pressure-plate (34a) configured to be pressed by a door of the liquid pump (21) and to press the pumping-tube segment (12b) against a pressure-sensor that is installed in the liquid pump (21);
installing the pumping tube-segment (12b) into the liquid pump (21);
**characterized by**,
the installing the pumping tube-segment (12b) into the liquid pump (21) further including installing a stretching-key (13) into a recess (21b) of the liquid pump (21), the stretching-key (13) including a collar clamped onto the pumping tube-segment (12b), wherein the distance between the identifying key (14) and the stretching key (13) corresponds to the respective distance between the niches (21a) and the recess (21b) in the liquid pump (21); and
tightening, holding straight and stretching the pumping tube-segment (12b) to a determined degree when both the identifying key (14) and the stretching key (13) are installed into the liquid pump (21).

2. The method of claim 1, wherein the distance between the identifying-key (14) and the stretching-key (13) ranges from 45 millimetres to about 47 millimetres.

3. The method of claim 1, wherein the stretching-key (13) is clamped near an end of the pumping tube-segment (12b).

4. The method of claim 1, wherein the stretching-key (13) is ring-shaped to allow detection of air in the pumping tube-segment (12b) by a liquid pump air detector through a hole in the stretching-key (13).

5. The method of claim 4, wherein the stretching-key (13) is configured to be disposed in front of the liquid pump air detector when the stretching-key (13) is installed in the liquid pump (21).

6. The method of claim 4, wherein the stretching-key (13) is configured to be disposed in back of the liquid pump air detector when the stretching-key (13) is installed in the liquid pump (21).

7. The method of claim 1, wherein the stretching-key (13) is configured to center the pumping tube-segment (12b) in front of an air sensor detector of the liquid pump (21) to ensure optimal air detection in the pumping tube-segment (12b).

8. The method of claim 1, wherein the pumping tube-segment (12b) has an inside diameter between 2 millimetres and 4 millimetres.

9. The method of claim 1, wherein the identifying-key (14) prevents the liquid pump (21) from starting operation unless the pressure-sensor measures a determined pressure.

10. The method of claim 1, wherein the pressure-sensor is installed in the liquid pump (21) behind the identifying-key (14).

11. The method of claim 1, wherein the identifying-key (14) is clamped near an end of the pumping tube-segment (12b).

## Patentansprüche

1. Verfahren zum Installieren eines Pumprohrsegments (12b) in einer Flüssigkeitspumpe (21), umfassend:
Bereitstellen des Pumprohrsegments (12b) mit einem darauf angeordneten Identifikationsschlüssel (14), wobei der Identifikationsschlüssel (14) einen Kragen beinhaltet, der auf dem Pumprohrsegment (12b) festgeklemmt ist, wobei der Identifikationsschlüssel (14) eine Anzahl an Zähnen (14a) aufweist, wobei jeder Zahn eine bestimmte Breite und eine bestimmte Position aufweist, wodurch ein Code erzeugt wird, der das Einsetzen des Pumprohrsegments (12b) nur in eine bestimmte Flüssigkeitspumpe (21) ermöglicht, die eine Reihe von Nischen (21a) aufweist, die nach demselben eindeutigen Strukturcode angeordnet sind, wobei der Identifikationsschlüssel (14) auch eine Druckplatte (34a) beinhaltet, die konfiguriert ist, um durch eine Tür der Flüssigkeitspumpe (21) gedrückt zu werden und um das Pumprohrsegment (12b) gegen einen Drucksensor zu drücken, der in der Flüssigkeitspumpe (21) installiert ist;
Installieren des Pumprohrsegments (12b) in der Flüssigkeitspumpe (21);
**dadurch gekennzeichnet, dass**
das Installieren des Pumprohrsegments (12b) in der Flüssigkeitspumpe (21) ferner das Installieren eines Spannschlüssels (13) in einer Aussparung (21b) der Flüssigkeitspumpe (21) beinhaltet, wobei der Spannschlüssel (13) einen Kragen beinhaltet, der auf dem Pumprohrsegment (12b) festgeklemmt ist, wobei der Abstand zwischen dem Identifikationsschlüssel (14) und dem Spannschlüssel (13) dem jeweiligen Abstand zwischen den Nischen (21a) und der Aussparung (21b) in der Flüssigkeitspumpe (21) entspricht; und
Anziehen, Geradehalten und Spannen des Pumprohrsegments (12b) bis zu einem bestimmten Grad, wenn sowohl der Identifikationsschlüssel (14) als auch der Spannschlüssel (13) in der Flüssigkeitspumpe (21) installiert sind.

2. Verfahren nach Anspruch 1, wobei der Abstand zwischen dem Identifikationsschlüssel (14) und dem Spannschlüssel (13) von 45 Millimetern bis etwa 47 Millimetern reicht.

3. Verfahren nach Anspruch 1, wobei der Spannschlüssel (13) nahe einem Ende des Pumprohrsegments (12b) festgeklemmt ist.

4. Verfahren nach Anspruch 1, wobei der Spannschlüssel (13) ringförmig ist, um Erfassung von Luft in dem Pumprohrsegment (12b) durch einen Flüssigkeitspumpenluftdetektor durch ein Loch in dem Spannschlüssel (13) zu ermöglichen.

5. Verfahren nach Anspruch 4, wobei der Spannschlüssel (13) konfiguriert ist, um vor dem Flüssigkeitspumpenluftdetektor angeordnet zu sein, wenn der Spannschlüssel (13) in der Flüssigkeitspumpe (21) installiert ist.

6. Verfahren nach Anspruch 4, wobei der Spannschlüssel (13) konfiguriert ist, um hinter dem Flüssigkeitspumpenluftdetektor angeordnet zu sein, wenn der Spannschlüssel (13) in der Flüssigkeitspumpe (21) installiert ist.

7. Verfahren nach Anspruch 1, wobei der Spannschlüssel (13) konfiguriert ist, um das Pumprohrsegment (12b) vor einem Luftsensordetektor der Flüssigkeitspumpe (21) zu zentrieren, um optimale Lufterfassung in dem Pumprohrsegment (12b) sicherzustellen.

8. Verfahren nach Anspruch 1, wobei das Pumprohrsegment (12b) einen Innendurchmesser zwischen 2 Millimetern und 4 Millimetern aufweist.

9. Verfahren nach Anspruch 1, wobei der Identifikationsschlüssel (14) verhindert, dass die Flüssigkeitspumpe (21) den Betrieb startet, es sei denn, der Drucksensor misst einen bestimmten Druck.

10. Verfahren nach Anspruch 1, wobei der Drucksensor in der Flüssigkeitspumpe (21) hinter dem Identifikationsschlüssel (14) installiert ist.

11. Verfahren nach Anspruch 1, wobei der Identifikationsschlüssel (14) nahe einem Ende des Pumprohrsegments (12b) festgeklemmt ist.

## Revendications

1. Procédé d'installation d'un segment de tube de pompage (12b) dans une pompe de liquide (21), comprenant :
la fourniture du segment de tube de pompage (12b) possédant une clé d'identification (14) disposée sur celui-ci, la clé d'identification (14) comprenant un collier qui est serré sur le segment de tube de pompage (12b), la clé d'identification (14) comportant un nombre de dents (14a), chaque dent possédant une largeur spécifique et un emplacement spécifique, créant ainsi un code qui permet l'insertion du segment de tube de pompage (12b) uniquement dans une pompe de liquide spécifique (21) qui possède un ensemble de créneaux (21a) qui sont agencés conformes au même code structurel unique, la clé d'identification (14) comprenant également un plateau de pression (34a) conçu pour être pressé par une porte de la pompe de liquide (21) et pour presser le segment de tube de pompage (12b) contre un capteur de pression qui est installé dans la pompe de liquide (21) ;
l'installation du segment de tube de pompage (12b) dans la pompe de liquide (21) ;
**caractérisé par**
l'installation du segment de tube de pompage (12b) dans la pompe de liquide (21) comprenant en outre l'installation d'une clé d'étirage (13) dans un évidement (21b) de la pompe de liquide (21), la clé d'étirage (13) comprenant un collier serré sur le segment de tube de pompage (12b), ladite distance entre la clé d'identification (14) et la clé d'étirage (13) correspondant à la distance respective entre les créneaux (21a) et l'évidement (21b) dans la pompe de liquide (21) ; et
le serrage, le maintien droit et l'étirage du segment de tube de pompage (12b) à un degré déterminé lorsque la clé d'identification (14) et la clé d'étirage (13) sont toutes deux installées dans la pompe de liquide (21).

2. Procédé selon la revendication 1, ladite distance entre la clé d'identification (14) et la clé d'étirage (13) étant comprise dans la plage allant de 45 millimètres à environ 47 millimètres.

3. Procédé selon la revendication 1, ladite clé d'étirage (13) étant serrée près d'une extrémité du segment de tube de pompage (12b).

4. Procédé selon la revendication 1, ladite clé d'étirage (13) étant en forme d'anneau pour permettre la détection d'air dans le segment de tube de pompage (12b) par un détecteur d'air de pompe de liquide à travers un trou dans la clé d'étirage (13).

5. Procédé selon la revendication 4, ladite clé d'étirage (13) étant conçue pour être disposée devant le détecteur d'air de pompe de liquide lorsque la clé d'étirage (13) est installée dans la pompe de liquide (21).

6. Procédé selon la revendication 4, ladite clé d'étirage (13) étant conçue pour être disposée à l'arrière du détecteur d'air de pompe de liquide lorsque la clé d'étirage (13) est installée dans la pompe de liquide (21).

7. Procédé selon la revendication 1, ladite clé d'étirage (13) étant conçue pour centrer le segment de tube de pompage (12b) devant un détecteur de capteur d'air de la pompe de liquide (21) pour assurer une détection d'air optimale dans le segment de tube de pompage (12b).

8. Procédé selon la revendication 1, ledit segment de tube de pompage (12b) possédant un diamètre intérieur compris entre 2 millimètres et 4 millimètres.

9. Procédé selon la revendication 1, ladite clé d'identification (14) empêchant le démarrage du fonctionnement de la pompe de liquide (21) à moins que le capteur de pression ne mesure une pression déterminée.

10. Procédé selon la revendication 1, ledit capteur de pression étant installé dans la pompe de liquide (21) derrière la clé d'identification (14).

11. Procédé selon la revendication 1, ladite clé d'identification (14) étant serrée près d'une extrémité du segment de tube de pompage (12b).
